# EUROPEAN PATENT APPLICATION

(11) **EP 0 857 779 A1**
(43) Date of publication of application: **12.08.1998**
(21) Application number: 97932844.0
(22) Date of filing: 24.07.1997
(51) Int. Cl.: C12N 1/21, C12N 9/02, C02F 3/34

(54) **PROCESS FOR THE BIODEGRADATION OF AROMATIC COMPOUNDS AND SYNTHESIS OF PIGMENTS AND COLORANTS, ALCALOIDS AND POLYMERS, WITH THE USE OF THE RECOMBINANT STRAIN ESCHERICHIA COLI P260**

(30) Priority: 26.07.1996 ES 9601666
(71) Applicant: UNIVERSIDAD COMPLUTENSE DE MADRID, E-28040 Madrid (ES)
(72) Inventor: MARTIN FERNANDEZ, Margarita, E-28040 Madrid (ES); FERRER MUNOZ, Estrella, E-28040 Madrid (ES); SANZ PERUCHA, Jesús, E-28040 Madrid (ES); GIBELLO PRIETO, Alicia, E-28040 Madrid (ES)
(74) Representative: Carpintero Lopez, Francisco
(86) International application number: ES9700189
(87) International publication number: WO9804679

(57) **Abstract**

The microbiological process for the biodegradation of aromatic compounds and synthesis of pigments and colourants, alkaloids and polymers, through the use of a recombinant strain *E. coli* P-260 is characterised by the constitutive expression of the enzyme 4-HPA hydroxylase of *K. pneumoniae*. This enzyme is involved in the degradation of 4-Hydroxyphenylacetic acid (4-HPA), one of the compounds present in black olive liquid ( 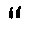 alpechin 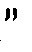 ). The low specificity of the substrate of the enzyme and its double catalytic activity: A) Hydroxylation of monohydroxylated compounds into hydroxylated intermediaries and B) oxydation of di-hydroxylated compounds to quinones, provide for its double use: 1) degradation of toxic aromatic compounds of the ambient environment and 2) synthesis of organic compounds of industrial interest, of which the structure base is a quinone.

## Description

### OBJECT OF THE INVENTION

The present invention lies in the field of Biotechnology and, specifically, in the area of biodegradation of toxic aromatic compounds and in that of the development of new methods for synthesizing pigments, alkaloids and polymers. The invention provides a "live system" for industrial application, based on the characteristics of the 4-hydroxyphenylacetate 3-hydroxylase enzyme. The *hpa* A and *hpa* H genes of *Klebsiella pneumoniae*, which encode for this enzyme have been cloned in the pUC18Not plasmid (Herrero et al., 1990; J. Bacteriol. 72:6557-67) and expressed effectively in E.coli. The use of the enzyme synthesized by recombinant bacteria is of special interest due to its double catalytic activity: the hydroxylation of monohydroxylated compounds into others that are dihydroxylated and the oxidation of the latter into quinones. The low specificity of the enzyme substrate allows a great variety of aromatic compounds to be transformed into dihydroxylated compounds which may be metabolized through the different biodegradation routes that exist in bacteria. This process leads to the decontamination of toxic or recalcitrant compounds in the environment, such as phenolic compounds of olive oil dregs, among which 4-hydroxyphenylacetate (4-HPA), the enzyme's preferred substrate, constitutes one of the major ones (Balice and Cera, 1984; Grasas y Aceites (Fats and Oils) 35:178-180). Moreover, in the absence of the concomitant catabolic routes, the oxidation of the dihydroxylated intermediate into quinone leads to the formation of cyclical organic structures of possible industrial interest: pigments similar to melanin, as well as to alkaloids and polymers.

### BACKGROUND OF THE INVENTION

Among the primary aims of all countries in the world is that of looking after and conserving the environment. As a consequence of industrial activity, many waste products are released into the environment which in most cases are difficult to degrade, toxic and/or recalcitrant. The massive accumulation of these waste products leads in the long term to the impoverishment of natural resources and to a breakdown of ecological balance. Most of the contaminating compounds contain a benzenic ring (aromatic compounds) in their structure and only microorganisms are able to metabolize this type of compounds.

The bacterium *K.pneumoniae* is found quite abundantly in soils (Deschamps et al., 1983; Ann. Microbiol. 134A: 189-196) and unlike other organisms, it is able to metabolize different aromatic compounds, including 4-hydroxyphenylacetic acid (4-HPA), one of the major aromatic compounds described in the composition of olive oil dregs (Balice and Cera, 1984; Grasas y Aceites (Fats and Oils), 35:178-180). Olive oil dregs are an industrial waste product originating in the manufacturing process of olive oil. Olive oil dregs cause serious environmental problems, owing to their high chemical demand for oxygen (C.D.O.) and biological demand for oxygen (B.D.O.). Moreover, the high phenolic content of these waste products gives them a high antimicrobial and phytotoxic activity.

An appropriate solution for the depuration of olive oil dregs could be the biotechnological application of microorganisms able to metabolize the toxic compounds of these waste products in activated pools or mud. With this purpose, it is necessary to have a deep knowledge of the catabolic routes used by the microorganisms for the different compounds of olive oil dregs and, in this way, to select the most appropriate species or "design" new strains that effectively degrade the wide variety of these substances.

4-HPA acid is formed as a product of microbial degradation of phenylacetic acid and of various aminoacids and phenolic amines. However, some microorganisms may metabolize this compound independently, both aerobically and anaerobically. Aerobic degradation of 4-HPA may be carried out through two alternative catabolic routes: the homogenistic acid route, in which 2,5-dihydroxyphenylacetate is formed as an intermediary compound; or the 3,4-dihydroxyphenylacetate route (Sparnins & Chapman, 1976; J. Bacteriol. 127: 362-366). In studies carried out in our laboratory, the degradation route of 4-HPA in *K pneumoniae* has been characterized: 4-HPA is transformed by the enzyme 4-HPA-3-hydroxylase into 3,4-dihydroxyphenylacetate (3,4-DHPA) and, after the action of a dioxygenase, the oxygenolitic splitting of the ring is produced, which allows total degradation of the compound into CO₂, succinate and pyruvate. These two enzymes are encoded by genes located in different operons and are induced in a different way (Martin and coll., 1991; J. Gen. Microbiol. 8: 199-203).

In previous work carried out in our laboratory, the cloning and expression in *Escherichia coli* was carried out of the genes that encode for the degrading enzymes which transform 3,4-DHPA into succinate semialdehyde (Martin et al., 1991; J. Gen. Microbiol. 8: 199-203). This patent puts forward the use of the *K. pneumoniae* genes which encode for the 4-HPA hydroxylase in decontamination processes of aromatic compounds or its application in the synthesis of polymers of different composition. In doing this, we base ourselves on the low specificity of this hydroxylase in relation to the aromatic substrate, and also on its double catalytic activity (hydroxylation of monohydroxylated compounds and oxidation of the dihydroxylates into quinones), which allows the use of carrier bacteria of this gene with two purposes:
A) Transformation of different aromatic compounds into others that are less toxic or easier to degrade.
B) Synthesis of new pigments, alkaloids and polymers of industrial use, owing to the high reactivity of the quinones formed.

### EXPLANATION AND DESCRIPTION OF THE INVENTION

The process that is the object of the patent consists in using the *E. coli* P260 strain (deposited in the Colección Española de Cultivos Tipo, {Spanish Collection of Standard Cultures}, Universidad de Valencia, Campus de Burjasot, 46100 BURJASOT, Valencia, with the access No. CECT 4627) and is characterized in that it constitutively expresses the 4-HPA hydroxylase of *K. pneumoniae*, for the biodegradation of aromatic compounds and the synthesis of pigments and colourants, alkaloids and polymers.

The pG260 plasmid is a carrier of the *hpa A* and *hpa H* genes of *K. pneumoniae*, which encode for the enzyme 4-HPA-3-hydroxylase which plays a part in the degradative route of 4-HPA, with which an *E. coli* (CC118) strain was transformed that did not contain the genes for the degradation of 4-HPA. By means of hydroxylation, this enzyme transforms 3-HPA and 4-HPA into 3,4-DHPA which, after the subdequent action of a dioxygenase, loses its aromaticity. For the isolation of these genes, the chromosomic DNA of *K. pneumoniae* was isolated and digested with the restriction enzyme Bam Hl. As a cloning vector the plasmid pUC18Not was chosen (Herrero et al., 1990; J. Bacteriol. 72:6557-67), digested with BamHl and dephosphorilated. Bonding mixtures were used in the transformation of the mutant strain W-21 of *E. coli* (Lacrec A), a mutant deficient in the 4-HPA hydroxylase enzyme. The transformants were select on plates that contained ampicillin (100 µg/ml), IPTG and X-Gal and subsequently on selected media with Ampicillin and 4-HPA as the sole source of carbon and energy. A recombinant plasmid complemented the 4-HPA mutation of *E. coli* W-21 and treating this transformant with acridin orange caused reversion to the mutant phenotype (due to loss of plasmids). These data indicated that the DNA inserts cloned in pUC18Not contained the genes that encode for 4-HPA hidroxylase of *K. pneumoniae*. Synthesis of the enzyme was checked by determining enzymatic activity in "standard" conditions, by means of a spectrophotometric test (Martin et al., 1991; J. Gen. Microbiol. 132: 621-628). In this way it was established that the enzyme synthesized constitutively in the strains of *E. coli* derived from K-12 (strains incapable of metabolizing 4-HPA because they lack all the genes of the degradative route), unlike what occurs in *K. pneumoniae* M5a1 or *E. coli* W, where synthesis of the enzyme is inducible by 3-HPA and/or 4-HPA. The *E. coli* K-12 (*E. coli* CC118) strains, transformed by the recombinant plasmid, were called *E. coli* P260. Table 1 shows the expression of the cloned 4-HPA hydroxylase in different defective mutations in this hydroxylase.

**Table 1**

| Expression of the cloned gene in various strains of *E. coli.* | | | |
|---|---|---|---|
| **STRAIN** | **SUBSTRATE GROWTH** | **4-HPA hidroxylase** | |
| | | **3-HPA** | **4-HPA** |
| *K. pneumoniae* M5a1 | 4-HPA | 26±9 | 30±5 |
| | LB | <1 | <1 |
| *E. coli* W-21 | Gly/4-HPA | <1 | <1 |
| *E. coli* W-21 (plasmid) | Gly/4-HPA/Ap | 28±5 | 23±6 |
| | LB/Ap | <5 | <5 |
| *E. coli* CC118 | Gly/4-HPA | <1 | <1 |
| *E. coli* P260 | Gly/4-HPA/Ap | 50±6 | 52±5 |
| | LB/Ap | 57±9 | 55±4 |

The *E. coli* P260 cultures produced a blackish brown pigmentation in LB or BHI as a consequence of the expression of the cloned genes. This fact suggested the existence in these media of a compound that could be transformed by the enzyme. To investigate this hypothesis, several aromatic compounds were analyzed, different from 3-HPA and 4-HPA, as possible substrates of the enzyme (Table 2).

**Table 2**

| Hidroxylase activity expressed by *E. coli* P260, on different aromatic compounds. | | | |
|---|---|---|---|
| **ORGANIC SUBSTRATE** | **λ MAX SUBSTRATE** | **% ACTIVITY** | **λ MAX PRODUCT** |
| 3-HPA | 274 nm | 100 | 279.8 nm |
| | 227 nm | | 203.4 nm |
| 4-HPA | 276 nm | 100 | 280 nm |
| | 223 nm | | 203.3 nm |
| 3,4-DHPA | 281 nm | 50 | 258 nm |
| | 203.3 nm | | 208.5 nm |
| 2-HPA | 275 nm | < 5 | 275 nm |
| | 222.6 nm | | 210 nm |
| 4-HBA | 245 nm | 15 | 250 nm |
| | 207.4 nm | | 205.7 nm |
| L-Tyr | 275 nm | 8 | 278 nm |
| | 220 nm | | 207.8 nm |
| L-Dopa | 278 nm | 10 | 475 nm |
| | 208 nm | | |
| 3,4-DHBA | 249.7 nm | 30 | 257 nm |
| | | | 208 nm |
| Catecol | 275 nm | 30 | 259 nm |
| | 210 nm | | |
| PA | 222 nm | < 5 | 222 nm |
| 2-HPA: 2-Hydroxyphenylacetate 4-HBA: 4-Hydroxybenzoate L-Tyr: L-Tyrosine L-Dopa: DL-Dihydroxyphenylalanin 3,4-DHBA: 3,4-Dihydroxybenzoate PA: Phenylacetic acid | | | |

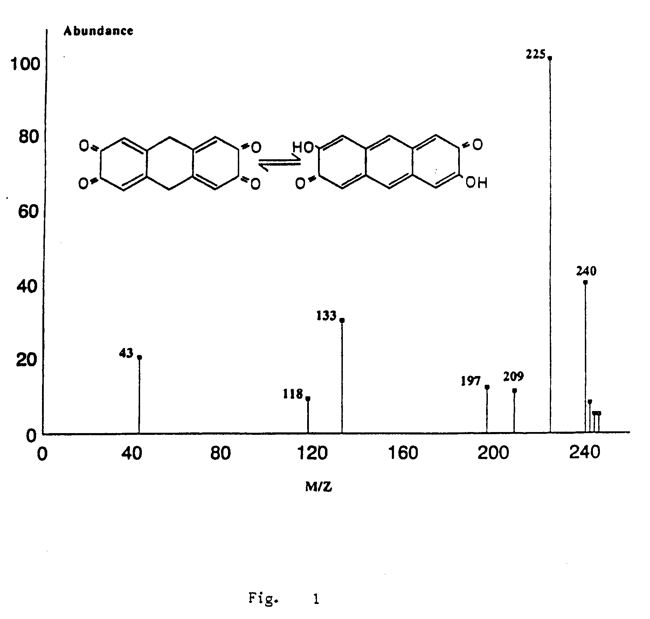

Unlike the isolated homologous enzyme of *Pseudomonas putida*
(Arunachalam et al., 1992; J. Biol. Chem. 267: 25848-25855), 4-HPA hydroxylase of *K. pneumoniae* displays a wide substrate tolerance in relation to the organic compound, and it is able to use some aromatic dihydroxylates such as 3,4-DHPA (Table 2). Moreover, the enzyme seems to require -OH groups in the carbons of positions 3- or 4- of the aromatic ring in order to recognize a compound as a sustrate, since 2-hydroxyphenylacetate (2-HPA), a structural analogue of 3-HPA or 4-HPA, is not transformed by the enzyme. As may be observed in Table 2, the products of reaction formed when L-Dopa and catecol are used as substrates, pesented maximum spectrophotometric absorbency, which coincides with that of dopaquinone and benzoquinone respectively. Moreover, the chromatographic mobility of the compounds obtained after the reaction was different from that of the initial substrates (data not shown). These results indicated that "in vitro" 4-HPA hydroxylase oxidated the dihydroxylated compounds into quinones. This fact was confirmed by analysis of the products of reaction (using spectrometry and gas chromatography), formed after a long period of reaction with 3,4-DHPA and 3,4-dihydroxybenzoate (3,4-DHBA) as substrates. As may be observed in Figure 1, in both cases a polymer was formed which had a cyclical structure as a repetitive unit, resulting from the fusion of two quinones. The said polymer did not come from the spontaneous oxidation of the 3,4-DHPA or 3,4-DHBA.

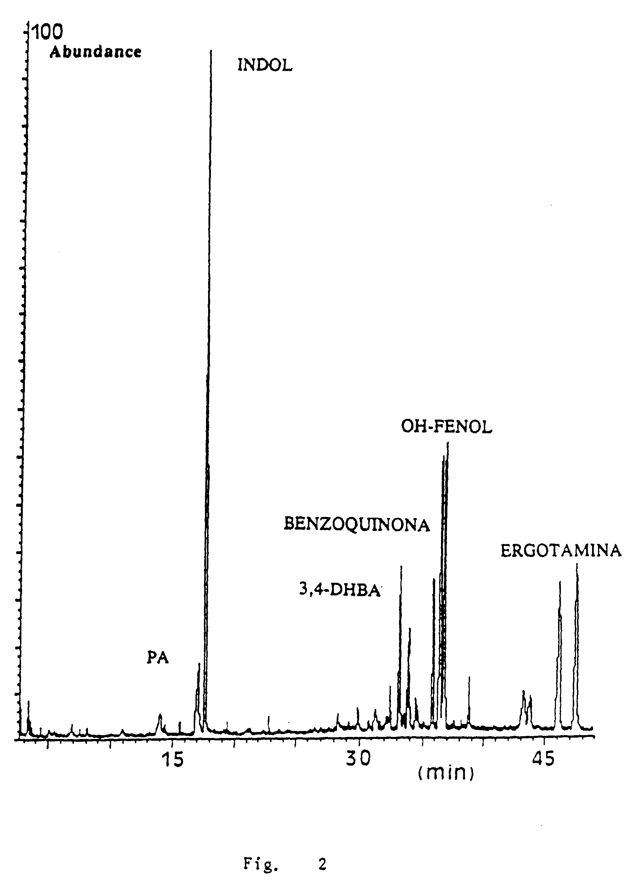

Moreover, the identification of the blackish-brown pigment of the *E. coli* P260 cultures also revealed the existence of a polymer similar to melanin, formed by indole and compounds derived from benzene (Figure 2). So, from the results obtained it may be deduced that 4-HPA hydroxylase of *K. pneumoniae* has an enzymatic activity similar to tyrosinase (Pomerantz and Murthy, 1974; Biochem. Biophys. 160: 73-82), but unlike this enzyme it does not have copper as a prosthetic group and displays a greater substrate tolerance. In this sense, it is an interesting fact that, although the existence of tyrosinase has been proved only in species of the genus *Vibrio spp*. and in Actinomycetes (Ivins and Holmes, 1980; Ann. Rev. Biochem. 51: 21-61; Yoshimoto et al., 1985; J. Biochem. 97; 1747-1754), the formation of pigments similar to melanin have been described in various groups of bacteria (*Aeromonas liquefaciens*, *A. salmonicida, Rhizobium phaseoli,* etc.) (Austard and Dahle, 1972; Acta Vet. Scand. 13: 251-259; Sadasivan and Neira, 1987; J. Bacteriol. 169; 1670-1677). From the results disclosed up to the pesent time the double catalytic activity of 4-HPA hydroxylase may be noted: 1) the use of a wide variety of mono-hydroxylated aromatic compounds as enzyme substrates and 2) the oxidation of the di-hydroxylated compounds into quinones. These two characteristics of 4-HPA hydroxylase allow its use in a dual rôle: on the one hand, in the biodegradation of toxic or recalcitrant aromatic compounds; and on the other, in the synthesis of pigments and colourants, alkaloids and polymers of industrial interest.

### DESCRIPTION OF THE DRAWINGS

Figure 1: Mass spectrum of the cyclical structure resulting from the fusion of two quinones.
Figure 2: Chromatogram of the pigment formed by the *E. coli* P260 cultures, where the formation of a polymer similar to melanin is shown.
Figure 3: Growth curve due to the degradation of 4-HPA in the *E. coli* W-P260 strain. (0) Bacterial growth, which includes the latent, exponential and stationary phases.
   (^{■}) Elimination of 4-HPA.

### DETAILED DESCRIPTION OF EMBODIMENT OF THE INVENTION

Below, by means of a laboratory test, without limiting its scope, an embodimente of the invention is described in the two applications set out above. The bacterium used in both cases was *E. coli* P260.

### A) Biodegradation of aromatic compounds:

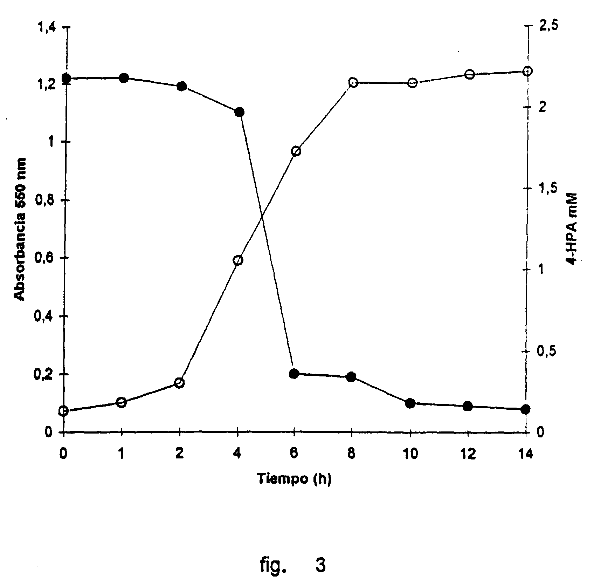

The genes cloned and expressed in *E. coli* demonstrated a high capacity for biotransforming aromatic compounds. In a test experiment, the following procedure was carried out: the *E. coli* W-P260 strain (obtained by transformation of the *E. coli* W-21 strain, mutant in hpa genes) was grown in a minimum PJC medium supplemented with glycerol and 4-HPA 2.5 mM (10% of which was marked with ¹⁴C in the aromatic ring). Incubation was carried out at 30°C and with stirring (200 rpm). In order to quantify the biotransformation of the aromatic ring, the CO₂ formed during the process was collected on NaOH 1N located in the lid of the matrass so that it would act as a trapping agent. Samples were taken at different times (Figure 3) so as to evaluate the formation of biomass (DO 680), release of CO₂ (cpm) and the disappearance of 4 HPA (measurement of concentration by HPLC). In Figure 3 it may be observed how the mineralization capacity of the *E. coli* W-P260 strain achieves mineralization of the aromatic compound in 12 hours.

Similar results are obtained using 3-HPA as a substrate. The low substrate specificity displayed by the enzyme (Table 2) extends its use in processes of biotransformation of aromatic compounds.

Another experience that demonstrates the utility for "biodegradation" purposes of the enzyme 4-HPA-3-hydroxylase, synthesized from the genes cloned in the recombinant plasmid that exists in *E. coli* P260, is its capacity for reverting various mutations in *Acinetobacter sp*. strains. The ADP6 mutants of *A. calcoaceticus* which are deficient in the enzyme catecol 1,2-dioxygenase (a strain produced by Ornston), are unable to metabolize benzoate (Stanier and Ornston, 1973; Adv. Microb. Physiol. 54: 1399-1404). However, the transformation of this mutant with the recombinant plasmids converts these mutants into bacteria capable of metabolizing benzoate.

### B) Synthesis of pigments, alkaloids and polymers.

As indicated above, different strains of *E. coli* derived from K-12 (eg. *E. coli* CC118, *E. coli* Jm109) produce a blackish-brown pigmentation of the BHI or LB media only when they are transformed with the recombinant plasmid. Therefore, this pigmentation is a consequence of the expression of the cloned *hpa* genes. As shown in Table 2, this enzyme has a wide substrate tolerance in relation to the aromatic compound and it is able to oxidate dihydroxylated compounds into quinones. On solid culture media (BHI/Ap), it could be observed that the pigmentation of the media increased when CuSO₄ or FeCI₃ were incorporated into the culture medium. Likewise, the addition of CuSO₄ and L-tyrosine (50 µg/ml) stimulated formation of the blackish pigmentation of the medium. Isolation and identification of the brownish-black pigment was carried out in the following way:

The *E. coli* P260 cells were grown aerobically (with stirring at 200 rpm/min) and at 37°C in LB until the culture reached maximum pigmentation of the medium (14 h.). The cells were then centrifuged (8000 rpm/min) and the supernatant was removed twice with ethyl acetate and evaporated until dry. The purified pigment displayed similar chemical characteristics to those described in the case of the melanins of different organisms: insolubility in water, solubility in methanol, ethanol and 1M NaOH, flocculation of the pigment by FeCI₃ and discolouration by H₂O₂ (Shieh and Maclean, 1974; Int. J. Biochem. 5: 643-647; Ogunnario and Hamilton-Miller, 1975; J. Med. Microbiol. 8: 199-203). Part of the sample was resuspended in ethyl acetate and analyzed by spectroscopy and mass chromatography. The identification of the pigment corresponded with a polymer formed by indole, phenolic derivatives, dibenzoquinones and a structure similar to ergotamine (Figure 2). These results suggest that the pigment is very similar to melanin. However, analysis of the enzyme by atomic absorption spectroscopy did not show the existence of copper bonded to the enzyme. The absence of copper as a prosthetic group of 4-HPA-3-hydroxylase discounts the possibility that this enzyme could be a type of tyrosinase (della Ciopa et al., 1990; Biotech. 8: 634-638).

The first experience that demonstrates the possible industrial application of this enzyme consisted of the formation of polymers of the semiladder type, using 3,4-DHPA and 3,4-dihydroxybenzoate (3,4-DHBA) as substrates of the enzyme. Reaction mixtures were prepared which contained 0,1M sodium/potassium phosphate buffer, pH 7.5; 3,4-DHPA (or 3,4-DHBA) 5 mM; NaOH 10 mM and 5 mg of the partially purified enzyme. The reaction mixtures were maintained at 30°C overnight and subsequently the products of reaction were extracted with ethyl acetate and evaporated until dry. The resulting solid was suspended in 0.5 ml of ethyl acetate and part of the samples were identified by spectroscopy and mass chromatography. In both cases similar chromatograms were obtained consistent with the existence of a polymer that contained a dibenzoquinone as a structural unit (Figure 1).

Polymers of the semiladder type have industrial utility in the manufacture of fibres, films and adhesives. However, the potential for using this enzyme may be greater if we consider that the wide tolerance of 4-HPA-3-hydroxylase makes possible the use of a great variety of substrates as precursors of substances of industrial interest. For example, from aromatic aminoacids (phenylalanin and tyrosine) new alkaloids could be synthesized, and from phenolic compounds, different types of polymers and pigments in general (Campbell, 1994; in "Introduction to Synthetic Polymers". Oxford University Press).

Thus, for example, the enzyme's reaction with certain phenolic alcohols, such as saligenin could give rise to the formation of polymers of the phenoplast type (Saunders, 1973; in "Organic Polymer Chemistry". John Wiley and Sons, Inc., New York). Also, the enzyme is able to produce, from LB, a pigment very similar to melanin, which could be used in cosmetics and for obtaining dyes and colourants.

## Claims

1. A microbiological process for the biodegradation of aromatic compounds and the synthesis of pigments and colourants, alkaloids and polymers, using the recombinant strain *E. coli* P260 (deposited in the CECT with the access No. CECT 4627), characterized in that it constitutively expresses the 4-HPA hydroxylase of *K. pneumoniae*.

2. A microbiological process for the biodegradation of aromatic compounds using the recombinant strain *E. coli* P260 as claimed in claim 1, characterized in that it includes:
A) Aromatic compounds derived from benzene with a -OH group in carbons 3- or 4- of the ring, and in particular, 4-hydroxyphenylacetic acid.
B) Mono- and dihydroxylated aromatic compounds.
C) Aromatic aminoacids and derivatives (with the characteristics of paragraph A).

3. A microbiological process for the biodegradation of aromatic compounds using the recombinant strain *E. coli* P260 as claimed in claims 1 and 2, characterized in that it includes compounds that contaminate the environment and, in particular, the elimination of the phenolic compounds of "olive oil dregs".

4. A microbiological process for the biodegradation of aromatic compounds using the recombinant strain *E. coli* P260 as claimed in the precedent claims, characterized in that it includes aromatic compounds present in herbicides, pesticides and industrial waste.

5. A process for the synthesis of organic compounds, as claimed in claims 1 and 2, characterized in that it includes dihydroxylated aromatic compounds and quinones.

6. A process for the synthesis of organic compounds of industrial interest, as claimed in claims 1 and 5, characterized in that it includes:
a) Pigments in general and, in particular, those that are similar to melanin, which are of use in cosmetics as dyes and colourants.
b) The obtaining of alkaloids.
c) The synthesis of substrate blocks for the preparation of new antibiotics derived from penicillin.
d) The production of polymers of the semiladder type or the phenoplast type.
